# EUROPÄISCHE PATENTSCHRIFT

(11) **EP 0 856 509 B1**
(45) Veröffentlichungstag und Bekanntmachung des Hinweises auf die Patenterteilung: **04.04.2001**
(21) Anmeldenummer: 98101386.5
(22) Anmeldetag: 27.01.1998
(51) Int. Cl.: C07C 231/08

(54) **Verfahren zur Herstellung von Diacylimiden**
Process for the preparation of diacylimides
Procédé de préparation de diacylimides

(30) Priorität: 31.01.1997 DE 19703549
(43) Veröffentlichungstag der Anmeldung: 05.08.1998
(73) Patentinhaber: Clariant GmbH, 65929 Frankfurt am Main (DE)
(72) Erfinder: Becherer, Johannes, Dr., 63477 Maintal (DE); Delpy, Klaus, Dr., 63128 Dietzenbach (DE); Keil, Karl-Heinz, Dr., 63454 Hanau (DE); Mees, Bernhard, Dr., 65817 Eppstein (DE)

(56) Entgegenhaltungen:
- WO-A-91/15474
- H. SASAKI ET AL : "Synthesis and anticonvulsant activity of 1-acyl-2-pyrrolidinone derivatives" JOURNAL OF MEDICINAL CHEMISTRY, Bd. 34, Nr. 2, 1991, Seiten 628-633, XP002082623 WASHINGTON US
- M. BODANSZKY ET AL: "Side reactions in peptide synthesis. II. Formation of succinimide derivatives from aspartyl residues" JOURNAL OF ORGANIC CHEMISTRY, Bd. 40, Nr. 17, 1975, Seiten 2495-2499, XP002082624 EASTON US
- T. TSUNODA ET AL : "An efficient method for hydrolysis of N-monosubstituted amides via acetoxypivalimides" TETRAHEDRON LETTERS, Bd. 31, Nr. 5, 1990, Seiten 731-734, XP002082625 OXFORD GB
- F. M. F. CHEN ET AL : "Diisopropylamine eliminates dipeptide formation during the acylation of amino acids using benzoyl chloride and some alkyl chloroformates" CANADIAN JOURNAL OF CHEMISTRY., Bd. 65, 1987, Seiten 1224-1227, XP002082626 OTTAWA CA

## Beschreibung

Die vorliegende Erfindung betrifft ein Verfahren zur Herstellung von Diacylimiden aus Säureamiden und Säurechloriden in Gegenwart von N,N-Diisopropyl-N-ethylamin.

EP-A-0 523 085 offenbart die Umsetzung von 3-Dimethylaminopropylamin in zwei Stufen mit Acetanhydrid zum N,N-Diacetyl-dimethylaminopropylamin. Während diese Reaktion zwar bei symmetrischem Diacetylimiden zum Erfolg führt, versagt sie jedoch bei der Herstellung von unsymmetrischen Diacetylimiden, da sie zur Umacetylierung führt, und damit zu unerwünschten symmetrischen Verbindungen.

Der Einsatz von Säurechloriden als Edukte ist literaturbekannt. So wird bei der als Schotten-Baumann-Reaktion bezeichneten Umsetzung ein Amid mit einem Säurechlorid in Gegenwart einer Base wie NaOH oder Soda in Wasser acyliert. Die Base wird benötigt, um die entstehende Salzsäure zu binden. Der Nachteil dieser Reaktion liegt in der Tatsache, daß bei hydrolyseempfindlichen Produkten die Ausbeute des gewünschten Zielproduktes sehr niedrig sein kann.

Tetrahedron Letters 31 (5), 731-734, 1990 offenbart die Umsetzung von Säurechloriden mit sekundären Amiden in Gegenwart von Triethylamin. Obwohl die Ausbeute mit über 90 % angegeben wird, so ist die Aufarbeitung von Triethylamin unter ökologischen und ökonomischen Gesichtspunkten unbefriedigend. Das gebildete Triethylammoniumhydrochlorid muß zur Wiedergewinnung von Triethylamin nach der Reaktion mit einer anorganischen Base (z.B. wäßrige Natronlauge) zersetzt werden. Wegen der guten Löslichkeit von Triethylamin und Wasser ineinander muß man eine aufwendige destillative Trennung der beiden Komponenten durchführen, welche dennoch eine relativ hohe Konzentration von Triethylamin im Abwasser nicht verhindern kann.

Ziel der vorliegenden Erfindung ist also ein Verfahren, bei dem selektiv und in hohen Ausbeuten sekundäre Säureamide mit Säurechloriden in Gegenwart einer Base umgesetzt werden, die sich ohne Umweltbelastung und ohne größere Kosten wieder regenerieren läßt.

Überraschenderweise wurde gefunden, daß bei Einsatz von N,N-Diisopropyl-N-Ethylamin (sog. Hünig-Base) das oben formulierte Ziel erreicht werden kann.

Gegenstand der Erfindung ist ein Verfahren zur Herstellung von Verbindungen der Formel 1 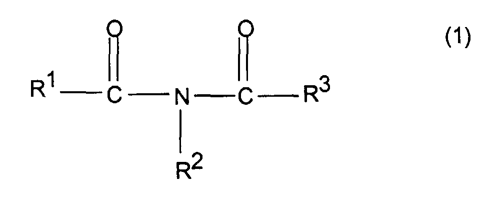 worin
R¹ und R² C₁-C₂₄-Alkyl oder C₂-C₂₄-Alkenyl bedeuten, oder worin R¹ und R² einen Ring mit 4 bis 8 Kohlenstoffatomen bilden, und R³ C₁-C₂₄-Alkyl, C₂-C₂₄-Alkenyl Aryl bedeutet, dadurch gekennzeichnet, daß man ein sekundäres Säureamidder der Formel 2 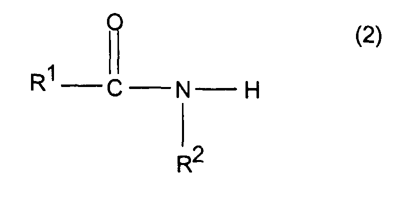 mit mindestens einem Säurechlorid der Formel 3 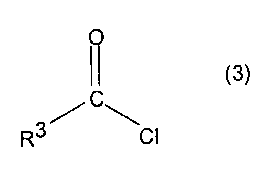 im Beisein von N,N-Diisopropyl-N-Ethylamin umsetzt,
wobei das Säurechlorid und das N,N-Diisopropyl-N-ethylamin in einem molaren Überschuß von jeweils bis zu 10 %, bezogen auf das sekundäre Amin, eingesetzt werden.

Bilden R¹ und R² einen Ring, dann kann dieser mit einem Rest R⁴ substituiert sein, wie in Formel 4 angegeben: 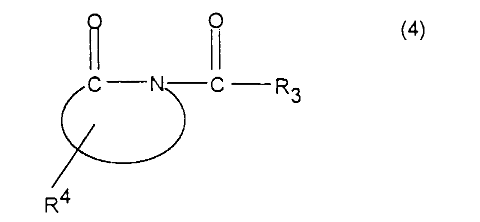 R⁴ kann C₁-C₄-Alkyl oder C₂-C₄-Alkenyl bedeuten.

R¹ und R² können unabhängig voneinander geradkettig oder verzweigt sein. Bevorzugt bedeuten R¹ und R² C₁-C₁₀-Alkyl oder C₂-C₁₀-Alkenyl, insbesondere C₈-C₁₀-Alkyl oder C₈-C₁₀-Alkenyl, für R² ist auch Methyl besonders bevorzugt. Bilden R¹ und R² einen Ring, so ist eine Ringgröße von 7 Atomen bevorzugt, was bedeutet, daß R¹ und R² zusammen 5 Kohlenstoffatome aufweisen.

R³ kann geradkettig oder verzweigt sein. R³ bedeutet bevorzugt C₁-C₁₂-Alkyl, C₆-C₁₄-Aryl oder -Aralkyl, insbesondere Benzyl oder Toluyl.

Die Reaktion kann sowohl mit geeigneten Lösungsmitteln wie Toluol, Xylol und/oder Tetralin durchgeführt werden, wie auch, vorzugsweise, ohne Lösungsmittel. Sowohl das Säurechlorid als auch das N,N-Diisopropyl-N-ethylamin werden in einem molaren Überschuß von jeweils bis zu 10 %, bezogen auf das sekundäre Amid eingesetzt.

Die Reihenfolge der Zugabe der Edukte ist beliebig, vorzugsweise wird das sekundäre Amid vorgelegt, und das Säurechlorid sowie die Base werden bei der benötigten Reaktionstemperatur gleichzeitig zugetropft. Die Reaktionstemperatur liegt üblicherweise zwischen 25 und 150°C, vorzugsweise zwischen 80 und 130°C.

Nach Ende der Reaktion erfolgt die Reinigung des Diacylimids durch Zugabe von soviel Wasser, daß das gebildete Hünig-Basenhydrochlorid in Lösung geht und das Imid über eine Phasentrennung bzw. Filtration getrennt wird. Das so erhaltene Diacylimid kann gegebenenfalls weiteren Reinigungsschritten (wie Destillation, Kristallisation) unterworfen werden. Die wäßrige Hünig-Basenhydrochloridlösung wird anschließend mit einer equimolaren Menge NaOH versetzt, wobei sich die Hünig-Base als Oberphase abscheidet und nach der Phasentrennung von der wäßrigen Unterphase separiert werden kann. Der Restwassergehalt in der Hünig-Base liegt dabei unter 0,15 %. Das ist überraschend, da das strukturverwandte Triethylamin fast unbeschränkt mit Wasser mischbar ist. Die so erhaltene Base kann entweder direkt oder nach einer eventuellen Reinigung (z.B. Destillation) wieder verwendet werden.

Diacylamide der beschriebenen Art können beispielsweise als Insektizide oder Bleichaktivatoren verwendet werden.

### Beispiel 1

In einem 4-l-Mehrhalskolben mit Rührer, Thermometer, Gaseinleitungsrohr, Rückflußkühler und 2 Tropftrichtern werden 1370,3 g (8 mol) Pelargonsäuremethylamid (Fp ca. 37°C) mit Stickstoff überlagert und aufgeschmolzen. Bei einer Innentemperatur von 85 - 95°C werden aus zwei Tropftrichtern parallel zueinander 690,8 g (8,8 mol) Acetylchlorid und 1116,7 g (8,64 mol) Hünig-Base im Verlaufe von 70 Minuten solcherweise zugetropft, daß das Acetylchlorid etwa 10 - 20 % schneller als die Hünig-Base dem Ansatz zugegeben wird. Dabei wird die Reaktion mittels Wasserbad gekühlt. Nach Beendigung der Zugabe wird 4 Stunden bei 85 - 90°C nachgerührt. Man erhält eine braune, gut rührbare Suspension von Hünig-Base-Hydrochlorid im Rohprodukt, welche unter Rühren auf Raumtemperatur abgekühlt wird. Dabei setzt sich ein Teil des Hydrochlorids am oberen Teil der Kolbenwand fest. Der Ansatz wird mit 1262 ml Wasser versetzt und eine halbe Stunde kräftig verrührt, wobei das Hünig-Base-Hydrochlorid komplett in Lösung geht. Man läßt absitzen (Dauer ca. Eine halbe Stunde) und saugt die untere, sauber abgetrennte wäßrige Phase ab. Der Ansatz wird mit weiteren 800 ml Wasser versetzt, kräftig verrührt und zur besseren Abtrennung in einen Scheidetrichter übergeführt. Man läßt ca. 1,5 Stunden absitzen und läßt die sauber abgetrennte unter wäßrige Phase ablaufen. Die organische Produktphase wird bei 5 bis 7 mbar aus einem 2-l-Dreihalskolben mit Rührer und Innenthermometer über eine 10-cm-Füllkörperkolonne mit Raschigringen 6 mm ohne Rücklauf destilliert. Zunächst wird bei einer Kopftemperatur von 30 bis 130°C ein Vorlauf abgenommen, dann destilliert das Produkt bei einer Kopftemperatur von 135 bis 143°C ab. Die Ölbadtemperatur liegt während des größten Teils des Destillation bei 155°C, am Ende wird die Badtemperatur bis auf 210°C erhöht und destilliert, bis nichts mehr übergeht. Der Destillationsrückstand ist auch nach dem Abkühlen auf Raumtemperatur dünnflüssig. Die Produktausbeute liegt bei 94 % und das so hergestellte N-Nonanyl-N-Acetylmethylimid hat eine Reinheit von 99 %.

### Wiedergewinnung der Hünig-Base

Die vereinigten wäßrigen Extrakte werden unter Rühren mit 800 g (10 mol) 50 %iger wäßriger Natronlauge alkalisch gestellt. Nach Abstellen des Rührers scheidet sich innerhalb von wenigen Minuten die Hünig-Base als obere honigbraune Schicht ab. Zwischen den beiden Schichten entsteht eine geringe Menge einer schwarzen organischen Masse, die weder in der wäßrigen Phase noch in Hünig-Base, aber in Aceton löslich ist. Die Phasen werden getrennt. Die wiedergewonnene Hünig-Base enthält 0,14 % Wasser und kann ohne weitere Aufarbeitung wieder eingesetzt werden.

### Beispiel 2 (Vergleichsbeispiel, nicht erfindungsgemäß)

Ersetzt man die in Beispiel 1 verwendete Hünig-Base durch 50 %ige NaOH und verfährt in gleicher Weise wie in Beispiel 1 angegeben, so enthält das Reaktionsgemisch vor der Aufarbeitung das gewünschte Produkt nur in einer Ausbeute von ca. 1 %.

### Beispiel 3

In einem 1-l-Vierhalskolben mit Rührer, Thermometer und Stickstoffüberleitung werden 200 g ε-Caprolactam vorgelegt und aufgeschmolzen. Unter Stickstoff werden nun innerhalb von 2 Stunden gleichzeitig 226 g N,N-Diisopropyl-N-Ethylamin (Hünig-Base) und 250 g Benzoylchlorid unter Rühren zugetropft. Die Temperatur wird anfangs bei 90°C gehalten und dann weiter auf 110°C gesteigert. Nach beendeter Reaktion wird die 80°C warme Schmelze auf 350 ml Wasser bei Raumtemperatur gegeben und verrührt. Nach einer Stunde wird das angefallene Benzoylcaprolactam abfiltriert und getrocknet. Man erhält das N-Benzoylcaprolactam in einer Ausbeute von 95 % und einer Reinheit nach GC > 99 %. Die Aufarbeitung der Hünig-Base erfolgt gemäß Beispiel 1. Auch hier kann das abgeschiedene N,N-Diisopropyl-N-Ethylamin (Wassergehalt: 0,13 %) ohne weitere Reinigung wieder eingesetzt werden.

### Beispiel 4 (Vergleichsbeispiel, nicht erfindungsgemäß)

Verglichen wird hierzu der Einsatz von Soda anstelle der Hünig-Base: 226,3 g e-Caprolactam, 159,0 g Natriumcarbonat und 1290 g Toluol werden in einem 4-l-Kolben vorgelegt. Bei Rückfluß werden innerhalb 2 h 281,1 g Benzoylchlorid so zugetropft, daß die CO₂-Entwicklung kontrolliert verläuft. Dann wird noch 4 h unter Rückfluß erhitzt. Die Reaktionsmischung wird mittels Wasserkühlung auf Raumtemperatur abgekühlt (Dauer ca. 15 min.), mit 500 g Wasser versetzt, kräftig gerührt und anschließend die wäßrige Phase in einem Scheidetrichter abgetrennt. Die verbleibende Toluolphase wird im Rotationsverdampfer bei einer Temperatur von 60°C eingeengt und Restmengen von Toluol im Vakuumtrockenschrank entfernt. Man erhält ein helles Öl, das beim Abkühlen kristallisiert. Smp.: 69°C. Man erhält das N-Benzoylcaprolactam in einer Ausbeute von 88 % und einer Reinheit von 98 %. Das entstandene CO₂ muß separat aufgefangen und entsorgt werden.

### Beispiel 5

In einem 1-l-Mehrhalskolben mit Rührer, Thermometer, Stickstoffüberleitungsrohr und 2 Tropftrichtern werden 137 g einer Mischung von C₈ / C₁₀ Fettsäureethylamid (Verhältnis 50:50) mit Stickstoff überlagert und auf 90°C unter Rühren erhitzt. Anschließend läßt man gleichzeitig 68 g Propionylchlorid und 111,7 g Hünig-Base im Verlauf von 1,5 h zutropfen. Die Reaktionswärme wird durch Kühlung abgeführt und man hält die Temperatur bei 90°C. Danach wird noch 5 Stunden nachgerührt und anschließend auf Raumtemperatur abgekühlt. Der Ansatz wird mit 130 ml Wasser versetzt und eine halbe Stunde kräftig verrührt, wobei das Hünig-Base-Hydrochlorid komplett in Lösung geht. Man läßt eine halbe Stunde absitzen und trennt dann von der wäßrigen Unterphase. Die Oberphase wird über eine kurze Füllkörperkolonne i.V. destilliert, wobei der Hauptlauf bei einer Kopftemperatur von 130 - 152°C übergeht (5 - 10 mbar). Die Produktausbeute liegt bei 95 % und die Produkteinheit des so hergestellten gemischten Diacylimids bei 99 %. Die Wiedergewinnung der Hünig-Base erfolgt wie in Beispiel 1 beschrieben.

## Patentansprüche

1. Verfahren zur Herstellung von Verbindungen der Formel 1 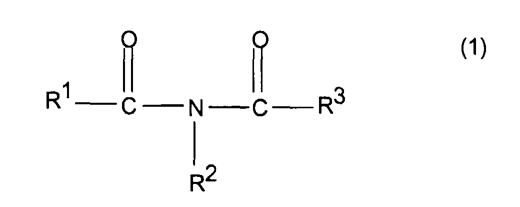 worin
R¹ und R² C₁-C₂₄-Alkyl oder C₂-C₂₄-Alkenyl bedeuten, oder worin R¹ und R² einen Ring mit 4 bis 8 Kohlenstoffatomen bilden, und R³ C₁-C₂₄-Alkyl, C₂-C₂₄-Alkenyl oder Aryl bedeutet, dadurch gekennzeichnet, daß man ein sekundäres Säureamid der Formel 2 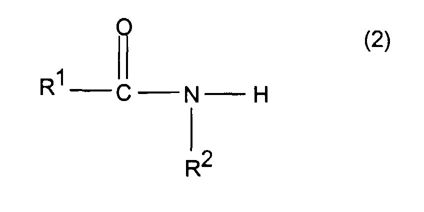 mit mindestens einem Säurechlorid der Formel 3 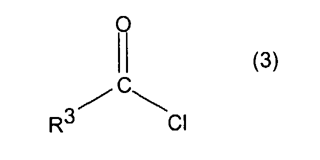 im Beisein von N,N-Diisopropyl-N-ethylamin umsetzt, wobei das Säurechlorid und das N,N-Diisopropyl-N-ethylamin in einem molaren Überschuß von jeweils bis zu 10 %, bezogen auf das sekundäre Amin, eingesetzt werden.

2. Verfahren nach Anspruch 1, dadurch gekennzeichnet, daß R¹ und R² unabhängig voneinander C₁-C₁₀-Alkyl, C₂-C₁₀-Alkenyl, insbesondere C₈-C₁₀-Alkyl oder C₈-C₁₀-Alkenyl bedeuten, wobei diese Reste geradkettig oder verzweigt sein können.

3. Verfahren nach Anspruch 1 und/oder 2, dadurch gekennzeichnet, daß R² Methyl bedeutet.

4. Verfahren nach einem der Ansprüche 1 bis 3, dadurch gekennzeichnet, daß R¹ und R² einen Ring mit einer Größe von 7 Atomen bilden.

5. Verfahren nach einem der Ansprüche 1 bis 4, dadurch gekennzeichnet, daß R³ geradkettiges oder verzweigtes C₁-C₁₂-Alkyl, C₆-C₁₄-Aryl oder -Aralkyl, insbesondere Benzyl oder Toluyl bedeutet.

6. Verfahren nach einem oder mehreren der Ansprüche 1, 4 und 5, dadurch gekennzeichnet, daß R¹ und R² miteinander einen Ring bilden, der mit einem Rest R⁴ substituiert ist, wobei R⁴ C₁-C₄-Alkyl oder C₂-C₄-Alkenyl bedeutet.

7. Verfahren nach einem oder mehreren der Ansprüche 1 bis 6, dadurch gekennzeichnet, daß die Reaktion ohne Lösungsmittel durchgeführt wird.

8. Verfahren nach einem oder mehreren der Ansprüche 1 bis 6, dadurch gekennzeichnet, daß die Reaktion in den Lösungsmitteln Toluol, Xylol, Tetralin oder Mischungen daraus durchgeführt wird.

9. Verfahren nach einem oder mehreren der Ansprüche 1 bis 8, dadurch gekennzeichnet, daß das sekundäre Amid vorgelegt wird und das Säurechlorid und das N,N-Diisopropyl-N-Ethylamin gleichzeitig zugetropft werden, wobei die Temperatur zwischen 25 und 150, vorzugsweise 80 und 130°C gehalten wird.

10. Verfahren nach einem oder mehreren der Ansprüche 1 bis 9, dadurch gekennzeichnet, daß die Reinigung des Diacylimids durch Zugabe von soviel Wasser erfolgt, daß das bei der Reaktion gebildete Hydrochlorid des N,N-Diisopropyl-N-Ethylamins in Lösung geht und von dem Diacylimid durch Phasentrennung getrennt wird.

## Claims

1. A process for the preparation of compounds of the formula 1 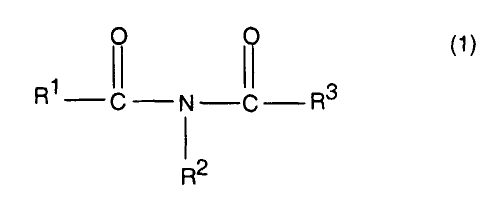 where
R¹ and R² are C₁-C₂₄-alkyl or C₂-C₂₄-alkenyl, or where R¹ and R² form a ring having 4 to 8 carbon atoms, and R³ is C₁-C₂₄-alkyl, C₂-C₂₄-alkenyl or aryl, which comprises reacting a secondary acid amide of the formula 2 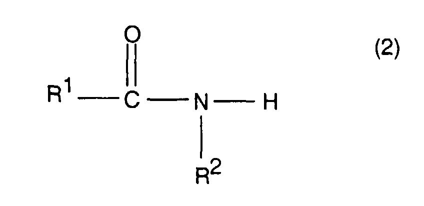 with at least one acid chloride of the formula 3 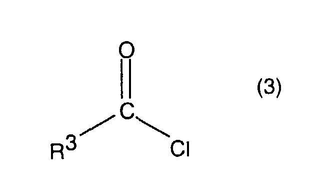 in the presence of N,N-diisopropyl-N-ethylamine, where the acid chloride and the N,N-diisopropyl-N-ethylamine are used in a molar excess of in each case up to 10%, based on the secondary amide.

2. The process as claimed in claim 1, wherein R¹ and R² independently of one another are C₁-C₁₀-alkyl, C₂-C₁₀-alkenyl, in particular C₈-C₁₀-alkyl or C₈-C₁₀-alkenyl, it being possible for these radicals to be linear or branched.

3. The process as claimed in claim 1 and/or 2, wherein R² is methyl.

4. The process as claimed in one of claims 1 to 3, wherein R¹ and R² form a ring containing 7 atoms.

5. The process as claimed in one of claims 1 to 4, wherein R³ is linear or branched C₁-C₁₂-alkyl, C₆-C₁₄-aryl or -aralkyl, in particular benzyl or toluyl.

6. The process as claimed in one or more of claims 1, 4 and 5, wherein R¹ and R² together form a ring which is substituted by an R⁴ radical, where R⁴ is C₁-C₄-alkyl or C₂-C₄-alkenyl.

7. The process as claimed in one or more of claims 1 to 6, wherein the reaction is carried out without solvent.

8. The process as claimed in one or more of claims 1 to 6, wherein the reaction is carried out in the solvents toluene, xylene, tetralin or mixtures thereof.

9. The process as claimed in one or more of claims 1 to 8, wherein the secondary amide is initially introduced and the acid chloride and the N,N-diisopropyl-N-ethylamine are added dropwise simultaneously, the temperature being maintained at between 25 and 150°C, preferably 80 and 130°C.

10. The process as claimed in one or more of claims 1 to 9, which comprises purifying the diacylimide by adding sufficient water to dissolve the hydrochloride of the N,N-diisopropyl-N-ethylamine formed during the reaction and separating it from the diacylimide by phase separation.

## Revendications

1. Procédé pour la préparation de composés de formule 1 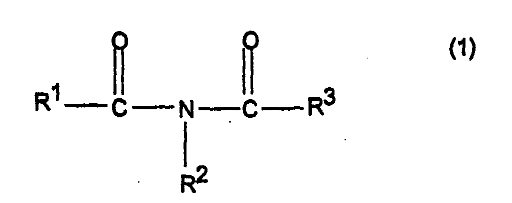 dans laquelle
R¹ et R² représentent un groupe alkyle en C₁ à C₂₄ ou alcényle en C₂ à C₂₄, ou dans laquelle R¹ et R² forment un cycle ayant de 4 à 8 atomes de carbone, et
R³ représente un groupe alkyle en C₁ à C₂₄, alcényle en C₂ à C₂₄ ou aryle,
caractérisé en ce que,
l'on met à réagir un amide d'acide secondaire de formule 2 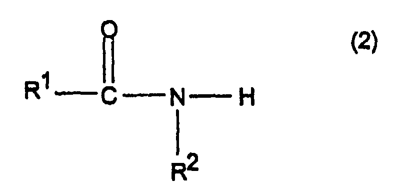 avec au moins un chlorure d'acide de formule 3 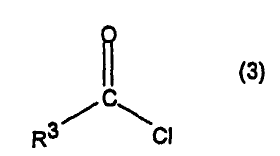 en présence de N,N-diisopropyl-N-éthylamine,
dans lequel on met en oeuvre le chlorure d'acide et la N,N-diisopropyl-N-éthylamine avec un excès molaire de respectivement 10% au maximum, par rapport à l'amine secondaire.

2. Procédé selon la revendication 1, caractérisé en ce que R¹ et R² représentent indépendamment l'un de l'autre un groupe alkyle en C₁ à C₁₀, alcényle en C₂ à C₁₀, en particulier alkyle en C₈ à C₁₀ ou alcényle en C₈ à C₁₀, dans lequel ces groupes peuvent être linéaires ou ramifiés.

3. Procédé selon la revendication 1 ou 2, caractérisé en ce que R² représente le méthyle.

4. Procédé selon l'une des revendications 1 à 3, caractérisé en ce que R¹ et R² représentent un cycle avec une taille de 7 atomes.

5. Procédé selon l'une des revendications 1 à 4, caractérisé en ce que R³ représente un groupe alkyle en C₁ à C₁₂ à chaîne linéaire ou ramifiée, aryle ou aralkyle en C₁ à C₁₂, en particulier benzyle ou toluyle.

6. Procédé selon une ou plusieurs des revendications 1, 4 et 5, caractérisé en ce que R¹ et R² forment ensemble un cycle qui est substitué avec un groupe R⁴, dans lequel R⁴ est un groupe alkyle en C₁ à C₄ ou alcényle en C₂ à C₄.

7. Procédé selon une ou plusieurs des revendications 1 à 6, caractérisé en ce que la réaction est effectuée sans solvant.

8. Procédé selon une ou plusieurs des revendications 1 à 6, caractérisé en ce que la réaction est effectuée dans les solvants toluène, xylène, tétraline ou des mélanges de ceux-ci.

9. Procédé selon une ou plusieurs des revendications 1 à 8, caractérisé en ce que l'on prépare l'amide secondaire et en ce qu'on verse ensuite goutte à goutte en même temps le chlorure d'acide et la N,N-diisopropyl-N-éthylamine, la température étant maintenue entre 25 et 150, de préférence entre 80 et 130°C.

10. Procédé selon une ou plusieurs des revendications 1 à 9, caractérisé en ce que la purification du diacyldiamide est réalisée par addition de suffisamment d'eau pour que le chlorhydrate de N,N-diisopropyl-N-éthylamine formé pendant la réaction passe en solution et se sépare du diacylimide par séparation de phase.
